# EUROPEAN PATENT APPLICATION

(11) **EP 1 345 149 A2**
(43) Date of publication of application: **17.09.2003**
(21) Application number: 02025418.1
(22) Date of filing: 14.11.2002
(51) Int. Cl.: G06F 19/00

(54) **Instructive-information supplying method and apparatus**

(30) Priority: 12.03.2002 JP 2002066566
(71) Applicant: Colin Corporation, Komaki-shi, Aichi-ken (JP)
(72) Inventor: Oka, Tohru, Komaki-shi, Aichi-ken (JP); Narimatsu, Kiyoyuki, Komaki-shi, Aichi-ken (JP)
(74) Representative: Winter, Brandl, Fürniss, Hübner, Röss, Kaiser, Polte Partnerschaft

(57) **Abstract**

A method of supplying, from a server which is connected via a communication line to a plurality of member's terminal devices which are respectively operated by a plurality of members who are registered in advance in the server, a set of instructive information based on a set of medical information related to one of the members and stored in the server, to the one member, in response to an instructive information request sent to the server from the one member, the method including an instructive-information-request identifying step of identifying the instructive-information request sent to the server from the one member, an instructor selecting step of selecting, when the instructive-information request sent from the one member has been identified, one of a plurality of instructors who are registered in advance in the server, and an information sending step of sending, to the selected instructor, the set of medical information related to the one member and stored in the server, to request the instructor to make the set of instructive information for the one member.

## Description

### BACKGROUND OF THE INVENTION

### Field of the Invention

The present invention relates to an instructive-information supplying method and an instructive-information supplying apparatus each for supplying instructive information appropriate to each of a plurality of members, such as instructive information for alimentotherapy or ergotherapy, in response to an instructive-information request sent from the each member.

### Related Art Statement

Instructive information with which a living person needs to comply to solve obesity, control hypertension, or improve athletic skill should be most appropriately prepared by an instructor who is specialized in the related field while the physical condition of the person is taken into consideration by the instructor.

However, conventionally, the instructive information has been prepared for general purposes or wide uses, i.e., for a number of persons. That is, the conventional instructive information has not been prepared for each person while the condition of each person is taken into consideration. Therefore, each person must modify, based on his or her own medical information such as weight or blood pressure, the general-purpose instructive information and apply the thus modified instructive information to himself or herself. However, each person may not have sufficient knowledge to be able to modify the general-purpose instructive information. If a person does not have the sufficient knowledge, or misunderstands the instructive information, then the person may erroneously modify the instructive information or apply the thus modified information to himself or herself.

### SUMMAEY OF THE INVENTION

It is therefore an object of the present invention to provide an instructive-information supplying method and an instructive-information supplying apparatus each for supplying, in response to a request from each member, instructive information suited for medical information of the each member.

The above object has been achieved by the present invention. According to a first aspect of the present invention, there is provided a method of supplying, from a server which is connected via a communication line to a plurality of member's terminal devices which are respectively operated by a plurality of members who are registered in advance in the server, a set of instructive information based on a set of medical information related to one of the members and stored in the server, to the one member, in response to an instructive-information request sent to the server from the one member, the method comprising an instructive-information-request identifying step of identifying the instructive-information request sent to the server from the one member, an instructor selecting step of selecting, when the instructive-information request sent from the one member has been identified, one of a plurality of instructors who are registered in advance in the server, and an information sending step of sending, to the selected instructor, the set of medical information related to the one member and stored in the server, to request the instructor to make the set of instructive information for the one member.

According to this invention, if, at the instructive-information-request identifying step, an instructive-information request sent from one of the members is identified, then, at the instructor selecting step, one of the pre-registered instructors is selected and, at the information sending step, the set of medical information related to the one member is sent to the instructor selected at the instructor selecting step, to request the instructor to prepare a set of instructive information for the member. Thus, the instructor can prepare the most suitable set of instructive information for the member, while taking the set of medical information of the member into consideration, and the most suitable set of instructive information is sent to the member.

According to a preferred feature of the first aspect of the present invention, the instructive-information supplying method further comprises a medical-information supplying step of selecting, from a plurality of sets of medical information respectively related to the plurality of members and stored in the server, the set of medical information related to a set of physical information of the one member inputted through a corresponding one of the member's terminal devices, and sending the selected set of medical information, to the one member, and an information storing step of storing the set of physical information of the one member inputted through the one member's terminal device and adding the set of physical information to the selected set of medical information.

According to this feature, the set of physical information of the member inputted through the member's terminal device to obtain the set of medical information related to the member, and the set of medical information supplied to the member are stored. Thus, the respective sets of physical information, and the respective sets of medical information, of the members are easily stored, on one hand; and, when each member requests a set of instructive information, the set of medical information including the set of physical information stored at the information storing step, is sent to the appropriate instructor, on the other hand. Thus, the more suitable set of instructive information for the requesting member is prepared, and is supplied to the member.

According to another feature of the first aspect of the present invention, the instructive-information supplying method further comprises a diagnosing step of making a diagnosis about a physical condition of the one member based on the set of physical information of the one member sent to the server from the one member's terminal device, and the information sending step comprises sending, to the selected instructor, the selected set of medical information including the made diagnosis.

According to this feature, if each member inputs his or her physical information to obtain his or her medical information, then, at the diagnosing step, a diagnosis is automatically made based on the physical information of the each member, and the thus made diagnosis is sent with the set of medical information to the appropriate instructor. Thus, the more appropriate set of instructive information for the member is prepared, and is sent to the member.

According to another feature of the first aspect of the present invention, the instructor selecting step comprises selecting the one of the instructors registered in advance in the server, based on a sort of the instructive information requested by the one member.

According to this feature, an appropriate instructor who has a specialized field corresponding to the sort of the instructive information requested by each member is selected. Thus, the more appropriate set of instructive information is supplied to the member.

According to a second aspect of the present invention, there is provided an instructive-information supplying apparatus including a server which is connected via a communication line to a plurality of member's terminal devices which are respectively operated by a plurality of members who are registered in advance in the server, and supplying, from the server, a set of instructive information based on a set of medical information related to one of the members and stored in the server, to the one member, in response to an instructive-information request sent to the server from the one member, the apparatus comprising an instructive-information-request identifying means for identifying the instructive-information request sent to the server from the one member; an instructor selecting means for selecting, when the instructive-information-request identifying means has identified the instructive-information request sent from the one member, one of a plurality of instructors who are registered in advance in the server; and an information sending means for sending, to the instructor selected by the instructor selecting means, the set of medical information related to the one member and stored in the server, to request the instructor to make the set of instructive information for the one member.

According to this invention, if the instructive-information-request identifying means identifies an instructive-information request sent from one of the members, then, the instructor selecting means selects one of the pre-registered instructors, and the information sending means sends the set of medical information related to the one member to the instructor selected by the instructor selecting means, so as to request the instructor to prepare a set of instructive information for the member. Thus, the instructor can prepare the most suitable set of instructive information for the member, while taking the set of medical information of the member into consideration, and the most suitable set of instructive information is sent to the member.

According to a preferred feature of the second aspect of the present invention, the instructive-information supplying apparatus further comprises a medical-information supplying means for selecting, from a plurality of sets of medical information respectively related to the plurality of members and stored in the server, the set of medical information related to a set of physical information of the one member inputted through a corresponding one of the member's terminal devices, and sending the selected set of medical information, to the one member; and an information storing means for storing the set of physical information of the one member inputted through the one member's terminal device and adding the set of physical information to the selected set of medical information.

According to this feature, the set of physical information of the member inputted to obtain the set of medical information related to the member, and the set of medical information supplied to the member are stored. Thus, the respective sets of physical information, and the respective sets of medical information, of the members are easily stored, on one hand; and, when each member requests a set of instructive information, the set of medical information including the set of physical information stored by the information storing means, is sent to the appropriate instructor, on the other hand. Thus, the more suitable set of instructive information for the requesting member is prepared, and is supplied to the member.

According to another feature of the second aspect of the present invention, the instructive-information supplying apparatus further comprises a diagnosing means of making a diagnosis about a physical condition of the one member based on the set of physical information of the one member sent to the server from the one member's terminal device, and the information sending means sends, to the selected instructor, the selected set of medical information including the made diagnosis.

According to this feature, if each member inputs his or her physical information to obtain his or her medical information, then, the diagnosing means automatically makes a diagnosis based on the physical information of the each member, and the information sending means sends the thus made diagnosis with the set of medical information, to the appropriate instructor. Thus, the more appropriate set of instructive information for the member is prepared, and is sent to the member.

According to another feature of the second aspect of the present invention, the instructor selecting means selects the one of the instructors registered in advance in the server, based on a sort of the instructive information requested by the one member.

According to this feature, an appropriate instructor who has a specialized field corresponding to the sort of the instructive information requested by each member is selected. Thus, the more appropriate set of instructive information is supplied to the member.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of an instructive-information supplying system as an instructive-information supplying apparatus utilizing a communication line, to which the present invention is applied;
Fig. 2 is a block diagram for explaining essential control functions of a server shown in Fig. 1;
Fig. 3 is a view showing an example of a set of medical information that is supplied from the server of Fig. 1 to a member;
Fig. 4 is a flow chart representing a portion of the essential control functions of the server of Fig. 1, i.e., a virtual-hospital controlling routine; and
Fig. 5 is a flow chart representing another portion of the essential control functions of the server of Fig. 1, i.e., an instructive-information-supply controlling routine.

### DETAILED DESCRIPTION OF PREFERRED EMBODIMENTS

Hereinafter, there will be described a preferred embodiment of the present invention in detail by reference to the drawings.

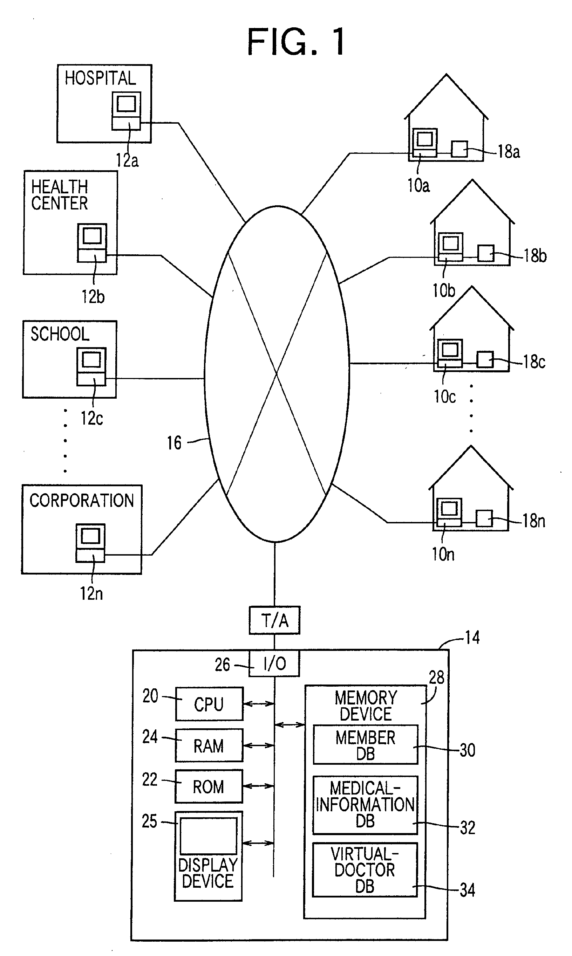

Fig. 1 is a view for explaining the construction of a medical-information and instructive-information supplying system as an instructive-information supplying apparatus to which the present invention is applied. As shown in Fig. 1, the present information supplying system includes a plurality of stationary-type or portable-type member's terminal devices 10a, 10b, 10c, ..., 10n that are operable by respective living persons as respective members. The member's terminal devices may be television sets, personal computers, or portable telephones that have the function of making communications via the internet. The information supplying system additionally includes a plurality of instructor's terminal devices 12a, 12b, 12c, ..., 12n that are operable by respective instructors and are provided in hospitals, health centers, schools, and corporations; and a server 14 as a virtual hospital that is provided in an information supplying company. The information supplying system further includes a communication line 16, such as wire or wireless internet (i.e., communication network) or wire or wireless telephone line, that connects the member's terminal devices 10n, the instructor's terminal devices 12n, and the server 14, with one another, so that highly secret codes, such as SSL, can be communicated among those elements 10n, 12n, 14. The member's terminal devices 10a, 10b, 10c, ..., 10n and the instructor's terminal devices 12a, 12b, 12c, ..., 12n are provided by, e.g., personal computers each including a display device, and the server 14 is provided by, e.g., a high-speed and high-capacity electronic computer.

A plurality of physical-information obtaining devices 18a, 18b, 18c, ..., 18n are connected to the plurality of member's terminal devices 10a, 10b, 10c, ..., 10n, respectively. Each of the physical-information obtaining devices 18 periodically obtains, from a corresponding one of the members (i.e., living persons), various sorts of physical information including blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes. Each of the physical-information obtaining devices 18 may include a plurality of sensors that detect the above-indicated various sorts of physical information, respectively, or an input device that is manually operable by a living person to input the above-indicated various sorts of physical information. The physical-information obtaining devices 18n supply the thus obtained respective sets of physical information to the member's terminal devices 10n, respectively. The blood pressure BP, e.g., systolic and diastolic blood pressure values, may be detected in such a manner that each of the physical-information obtaining devices 18n employs an inflatable cuff, the cuff is wound around an upper arm of the corresponding living person, and blood pressure values of the person are determined, according to so-called oscillometric method, based on change of respective amplitudes of respective heartbeat-synchronous pulses of a cuff pulse wave detected as a pressure signal by a pressure sensor from the cuff during changing of the pressing pressure of the cuff. Alternatively, the blood pressure may be obtained in such a manner that blood pressure values measured using a common sphygmomanometer from the person are inputted through operation of keys of an input device. The weight W may be detected in such a manner that each of the physical-information obtaining devices 18n employs a weight sensor, and a weight of the person is detected by the weight sensor, or alternatively the weight W may be obtained in such a manner that a weight measured using a common scales from the person is inputted through operation of keys of an input device. The heart rate HR may be calculated based on the number of heartbeat-synchronous pulses of the above-described cuff pulse wave that are produced in unit time, or based on the number of heartbeat-synchronous pulses of an electrocardiograph waveform that are produced in unit time and are detected by an electrocardiograph device, not shown. The electrocardiograph waveform ECG may be detected by an electrocardiograph device, not shown. The temperature TB may be detected by a temperature sensor, not shown, that is supported by the above-described cuff and is worn with the cuff on the person. The autonomic-nerve activity may be determined based on fluctuations of the heart rate values HR or the blood pressure values BP. The pulse-wave propagation velocity PWV may be determined based on a propagation time from the time when a second heart sound II is detected by a heart-sound microphone to the time when a dicrotic notch of a carotid pulse wave is detected, and a propagation distance between the heart and the carotid artery. The augmentation index AI may be defined as a ratio or proportion of one of an amplitude of an incident-wave component of an arterial pulse wave, such as carotid pulse wave, and an amplitude of a reflected-wave component of the pulse wave, to the other, and may be determined based on the waveform of the carotid pulse wave, or the cuff pulse wave.

The server 14 is essentially provided by a so-called microcomputer including a CPU (central processing unit) 20, a ROM (read only memory) 22 that stores control programs, a RAM (random access memory) 24 that functions as a temporary-storage device, a display device 25, and an input-and-output interface 26 that is connected via a terminal adaptor TA to the communication line 16. The server 14 includes a memory device 28 that stores various sorts of data bases (DB) corresponding to various sorts of information. More specifically described, the memory device 28 includes a member DB (data base) 30 that stores respective names of the living persons who have contracted, at their own charge or no charge, with the information supplying company to become, in advance, members who are to be supplied with medical information from the company; respective identification codes ID of the members; respective pass codes of the members; and respective names and identification codes ID of the instructors. The memory device 28 additionally includes a medical-information DB (data base) 32 that stores respective sets of medical information including respective sets of physical information sent from the members, and respective medical evaluations or diagnoses automatically made based on those sets of physical information. The memory device 28 further includes a virtual-doctor DB (data base) 34 that stores a diagnosing program for use in automatically making those medical evaluations or diagnoses on the sets of physical information or respective changes (or trends) of the sets of information. The virtual-doctor DB 34 additionally stores sets of instructive information, such as alimentotherapy program or ergotherapy program, that are made by the instructors based on the sets of physical information or the respective changes or trends of the sets of information so as to treat hypertension or obesity.

Thus, the member's terminal devices 10n are operated by the respective members who are registered in the server 14, and each one of the member's terminal devices 10n periodically sends a set of physical information of a corresponding one of the members to the server 14. At that time, the server 14 stores the set of physical information, and automatically makes, according to the diagnosing program stored in the virtual-doctor DB 34, a medical evaluation or diagnosis on the set of physical information or the change or trend of the current set of physical information from the past sets of physical information stored in the medical-information DB 32. In addition, the server 14 sends the thus made medical evaluation or diagnosis, together with the current and past sets of physical information of the member, to the member's terminal device 10n, so that the medical evaluation or diagnosis and the sets of physical information are displayed on the display device of the terminal device 10n of the member. In addition, each of the member's terminal devices 10n may be operated by a corresponding one of the members to request the server 14 to send one or more sets of instructive information, such as an instructive program to decrease weight or an instructive program to lower blood pressure. At that time, the server 14 selects, from the instructors who are registered in the server 14 and have respective specialized fields, the most appropriate instructor corresponding to the sort or sorts of the instructive information requested by the member, and sends, to the terminal device 12n of the selected instructor, the set of medical information related to the member and stored in the medical-information DB 32, so that the selected instructor can make a set of instructive information suitable for the member. The set of instructive information made by the selected instructor and received by the server 14 is sent to the terminal device 10n of the member who has requested, so that the set of instructive information is displayed on the terminal device 10n.

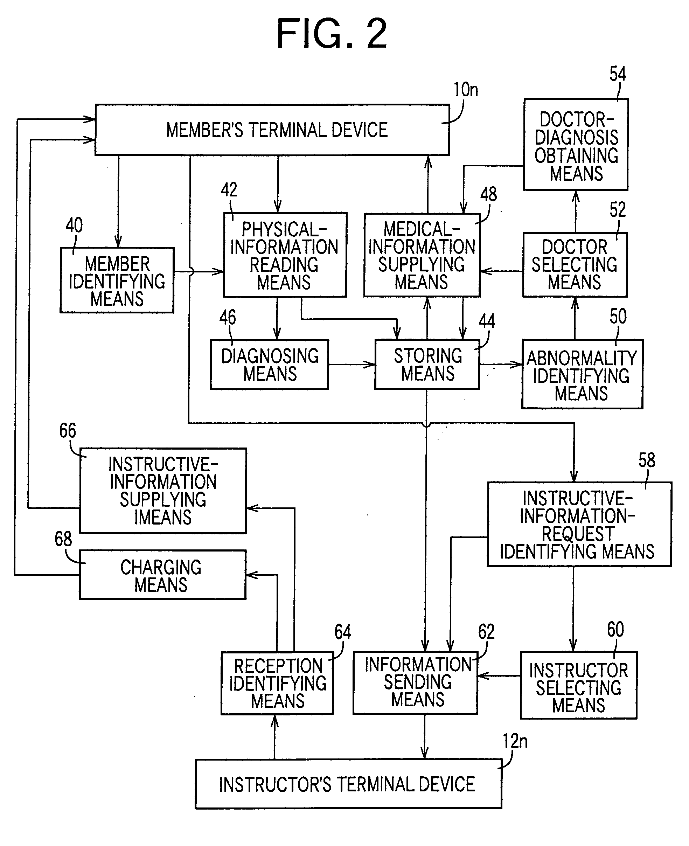

Fig. 2 is a block diagram for explaining the essential control functions of the above-described server 14. A member identifying means 40 judges whether an identification code ID inputted and sent by an arbitrary one of the member's terminal devices 10n is identical with one of the identification codes ID pre-stored in the member DB 30, and thereby judges whether a living person who has made access to the server 14 is one of the members pre-registered in the server 14. A physical-information reading means 42 reads, when the member identifying means 40 has identified the person as one of the registered members, the set of physical information sent by the member's terminal device 10n, and a storing means 44 stores, in the medical-information DB 32, the thus read set of physical information in such a manner that the set of physical information read is associated with the identification code ID of the member identified. A diagnosing means 46 automatically makes a diagnosis on the set of physical information read by the reading means 42, according to the diagnosing program pre-stored in the virtual-doctor DB 34. For example, the diagnosing means 46 judges whether the set of physical information read by the reading means 42 falls in a predetermined reference range, and thereby makes a medical evaluation or diagnosis. The storing means 44 stores, in the medical-information DB 32, the thus made medical evaluation or diagnosis such that the medical evaluation or diagnosis is associated with the identification code ID or the set of physical information. A medical-information supplying means 48 supplies, to the member's terminal device 10n that has sent the current set of physical information, the current and past sets of physical information, and the medical evaluation or diagnosis made on those sets of physical information, all of which have been stored by the storing means 44 in the medical-information DB 32, so that the trend of physical information and the medical evaluation or diagnosis are displayed on the member's terminal device 10n.

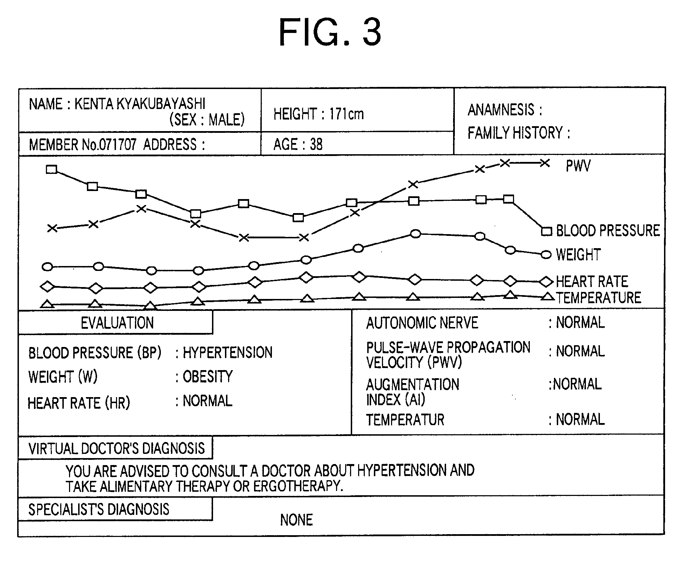

An abnormality identifying means 50 judges whether each set of physical information read by the reading means 42, or an amount of change of the set of physical information, falls outside a predetermined normal range and thereby judges whether each member is abnormal. The normal range may be identical with the above-described reference range. A doctor selecting means 52 selects, when the abnormality identifying means 50 judges based on the set of physical information that the member is abnormal, the most appropriate one of the doctors who are listed and stored in the memory device 28, based on the sort of the physical information judged as abnormal, and an address of the abnormal member, such that the selected doctor is specialized in the sort of the abnormality and is near to the address of the abnormal member. A doctor-diagnosis obtaining means 54 sends the current set of physical information judged as abnormal, and the past sets of physical information and the automatic evaluation or automatic diagnosis stored in the medical-information DB 32, to the doctor selected by the doctor selecting means 52, requests the doctor to make a diagnosis on the current set of physical information, and receives the diagnosis made by the doctor. The storing means 44 stores, in the medical-information DB 32, the diagnosis made by the doctor, such that the diagnosis is associated with the identification code ID or the physical information, and the medical-information supplying means 48 sends the diagnosis to the member. Fig. 3 shows an example of a set of medical information that is displayed on the display device of the member's terminal device 10n that has sent the set of physical information to the server 14. In this way, each member can send his or her own physical information via the member's terminal device 10n, so as to obtain the automatically made diagnosis and additionally obtain, when the physical information is abnormal, the diagnosis made by the actual doctor on the physical information. Thus, the member can go to the "virtual" hospital to take the medical examination, and even if the member may live at a remote place from the virtual hospital or the server 14, the member can obtain the results as if he or she had take an actual examination at an actual hospital.

An instructive-information-request identifying means 58 identifies an instructive-information request sent to the server 14 from an arbitrary one of the user's terminal devices 10n. An instructor selecting means 60 selects, when the instructive-information-request identifying means 58 has identified an instructive-information request sent from one of the user's terminal devices 10n, an appropriate one of the pre-registered instructors, based on the sort of the instructive information requested by the corresponding member, and the set of medical information pre-stored for the member in the medical-information DB 32. An information sending means 62 sends, to the terminal device 12n of the instructor selected by the instructor selecting means 60, information to request the selected instructor to make a set of instructive information, and additionally sends the set of medical information (including the automatic diagnosis) pre-stored for the member in the medical-information DB 32.

A reception judging means 64 judges whether the server 14 has received the set of instructive information sent from the terminal device 12n of the instructor. An instructive-information supplying means 66 supplies, to the terminal device 10n of the member who has requested, the set of instructive information the reception of which from the instructor has been judged by the reception judging means 64. Simultaneously, a charging means 68 charges the member who has requested the supply of the instructive information, for the use of the instructive information. To this end, the charging means 68 send a set of charging information to the user's terminal device 10n. The charging information may be a bill, or a notice to draw money from a bank account of the member who contracted with the information supplying company to allow it.

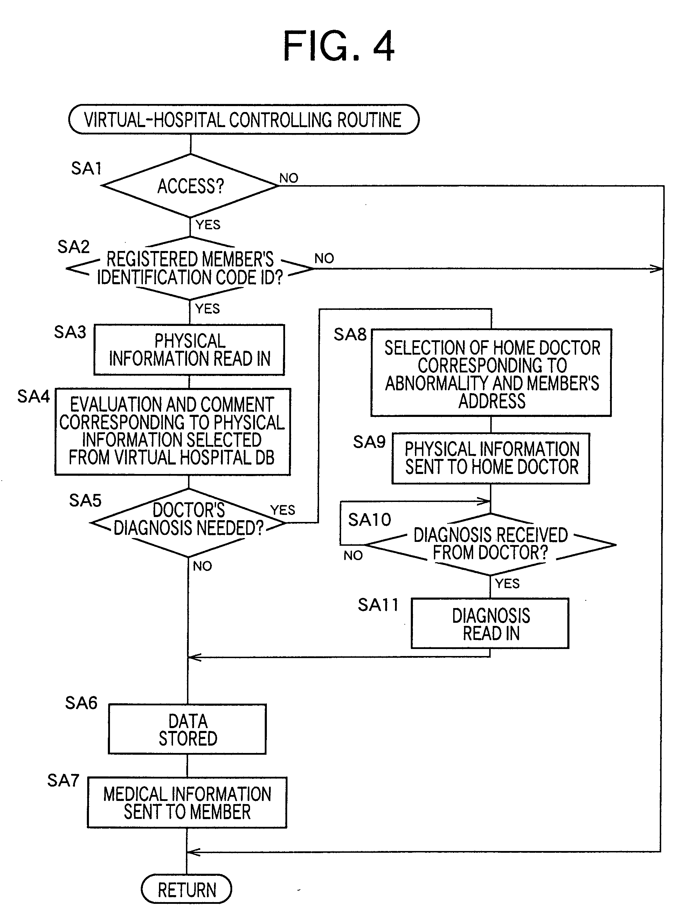

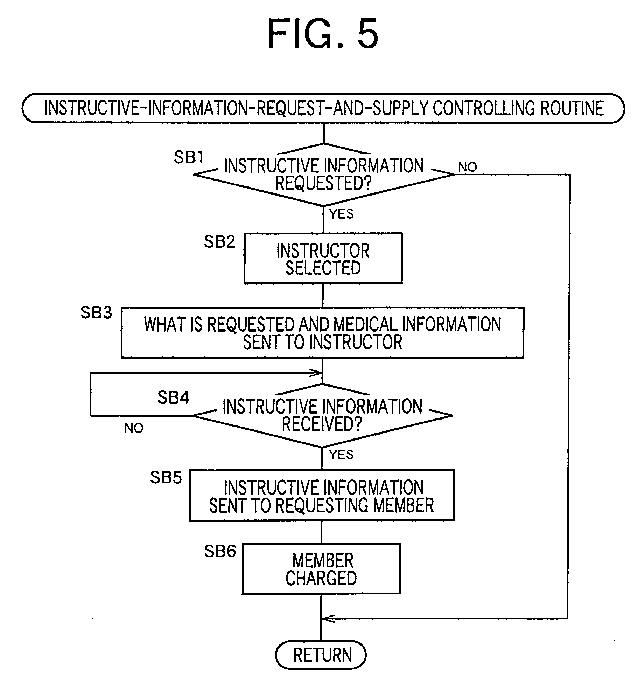

Figs. 4 and 5 show respective flow charts representing the essential control functions of the above-described server 14, i.e., Fig. 4 is a virtual-hospital (i.e., medical-information-supply) controlling routine and Fig. 5 is a instructive-information-supply controlling routine. These routines are iteratively executed at a short period.

At Step SA1 of Fig. 4 (hereinafter, "Step(s)" is omitted), the server 14 judges whether any one of the member's terminal devices 10n has made access to the server 14. If a negative judgment is made at SA1, this routine is quitted. On the other hand, if a positive judgment is made at SA1, the control goes to SA2 corresponding to the member identifying step or the member identifying means 40. At SA2, the server judges whether the member's identification code ID inputted by the member's terminal device 10n is identical with one of the member's identification codes ID pre-registered in the server. If a negative judgment is made at SA2, this routine is quitted. On the other hand, if a positive judgment is made at SA 2, the control goes to SA3 corresponding to the physical-information reading step or the physical-information reading means 42. At SA3, the server reads a set of physical information that has been obtained by the physical-information obtaining device 18n connected to the member's terminal device 10n and has been sent thereto from the terminal device 10n, i.e., blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes.

Then, the control goes to SA4 corresponding to the diagnosing step or the diagnosing means 46. At SA4, the server automatically makes, according to the automatically evaluating and diagnosing program pre-stored in the virtual-doctor DB 34, a diagnosis on the new set of physical information, the past set or sets of physical information, and the change of the new and past sets of physical information. For example, the server selects one of a plurality of evaluations and/or comments that corresponds to the new set of physical information. Fig. 3 shows the thus made evaluations and the thus made virtual doctor's diagnosis. Then, the control goes to SA5 corresponding to the abnormality identifying step or the abnormality identifying means 50. At SA5, the server judges whether it is needed to consult an actual doctor or a specialist. More specifically described, the server judges whether the new set of physical information falls outside a predetermined normal range. If a negative judgment is made at SA5, the control goes to SA6 corresponding to the storing step or the storing means 44. At SA6, the server stores, in the medical-information DB 32, the new set of physical information and the automatically made evaluation and/or diagnosis, such that the set of medical information is associated with the identification code ID of the member. Then, the control goes to SA7 corresponding to the medical-information supplying step or the medical-information supplying means 48. At SA7, the server sends, to the member's terminal device 10n that has sent the new set of physical information, the set of medical information that has been stored, for the member, in the medical-information DB 32, that is, the new and past sets of physical information (i.e., the trend of physical information) and the automatic evaluation and/or diagnosis, so that the set of medical information is displayed on the display device of the member's terminal device 10n, as shown in Fig. 3. Thus, each member can easily and economically request the virtual hospital or doctor to check his or her physical condition or make a diagnosis on it.

On the other hand, if a positive judgment is made at SA5, the control goes to SA8 corresponding to the doctor selecting step or the doctor selecting means 52. At SA8, the server selects, from the pre-stored list of doctors, a doctor or a specialist who is appropriate for the sort of the abnormal physical information, the degree of abnormality of the physical information, and the address of the member. For example, a specialist who is specialized in the sort of the abnormal physical information, or a doctor belonging to a medical institution located in an area where the member can go. Next, the control goes to SA 9 to SA11 corresponding to the doctor-diagnosis obtaining step or the doctor-diagnosis obtaining means 54. First, at SA9, the server sends, to the selected doctor, the abnormal, new set of physical information of the member and the past sets of physical information of the same, and requests the doctor to make a diagnosis on the sets of physical information. Then, at SA10, the server judges whether the server has received a diagnosis sent from the doctor. SA10 is repeated until a positive judgment is made. When a positive judgment is made at SA10, the control goes to SA11 to read in the diagnosis sent from the doctor. Subsequently, the control goes to SA6 and SA7. At SA6, the server stores, in the medical-information DB 32, not only the new set of physical information and the automatic evaluation or diagnosis, but also the diagnosis made by the actual doctor, such that the set of medical information is associated with the identification code ID of the member. Then, the control goes to SA7 corresponding to the medical-information supplying step or the medical-information supplying means 48. At SA7, the server sends, to the member's terminal device 10n that has sent the new set of physical information, the set of medical information that has been stored, for the member, in the medical-information DB 32, that is, the new and past sets of physical information (i.e., the trend of physical information), the automatic evaluation or diagnosis, and the diagnosis made by the actual doctor, so that the set of medical information is displayed on the member's terminal device 10n, as shown in Fig. 3, and additionally the diagnosis made by the actual doctor, and the name and address of the hospital to which the doctor belongs, or the address of the doctor are indicated in an area prepared therefor (i.e., the lowermost area). Thus, each member can easily and economically request the virtual hospital or doctor to check his or her physical condition or make a diagnosis on it, and additionally can obtain a diagnosis made by the actual doctor when the new set of physical information is judged as abnormal. If necessary, the member can request the doctor to re-examine his or her physical condition.

Then, the control goes to SB1 of Fig. 5 corresponding to the instructive-information-request identifying step or the instructive- information-request identifying means 58. At SB1, the server judges whether an arbitrary one of the respective user's terminal devices 12n has requested the server 14 to send a set of instructive information such as an instructive program for alimentotherapy or an instructive program for ergotherapy. If a negative judgment is made at SB1, this routine is quitted. On the other hand, if a positive judgment is made at SB1, the control goes to SB2 corresponding to the instructor selecting step or the instructor selecting means 60. At SB2, the server selects, from the pre-registered instructors, an appropriate instructor who is specialized in the sort of the instructive information requested by the corresponding member and the set of medical information pre-stored for the member. Then, the control goes to SB3 corresponding to the information sending step or the information sending means 62. At SB3, the server sends, to the terminal device 12n of the instructor selected at SB2, information to request the selected instructor to make a set of instructive information, and additionally sends the set of medical information (including the automatic diagnosis) pre-stored for the member in the medical-information DB 32.

Then, the control goes to SB4 corresponding to the reception judging step or the reception judging means 64. At SB4, the server judges whether the server has received the set of instructive information sent from the terminal device 12n of the instructor. Step SB4 repeated until a positive judgment is made. Then, the control goes to SB5 corresponding to the instructive-information supplying step or the instructive-information supplying means 66. At SB5, the server supplies, to the terminal device 10n of the member who has requested, the set of instructive information the reception of which from the instructor has been judged at SB4. Next, the control goes to SB6 corresponding to the charging step or the charging means 68. At SB6, the server sends charging information to the member's terminal device 10n, so as to charge the member who has requested the supply of the instructive information.

As is apparent from the foregoing description of the illustrated embodiment, when the instructive-information-request identifying step SB1 or the instructive-information-request identifying means 58 identifies an instructive-information request sent from one of the pre-registered members, the instructor selecting step SB2 or the instructor selecting means 60 selects one of the pre-registered instructors, and the information sending step SB3 or the information sending means 62 sends, to the instructor selected at the instructor selecting step SB2 or by the instructor selecting means 60, the set of medical information related to the one member and requests the instructor to make a set of instructive information for the member. Thus, the instructor can make the most appropriate set of instructive information for the member, while taking the set of medical information of the member into consideration, and the most appropriate set of instructive information is sent to the member.

In addition, in the illustrated embodiment, the medical-information supplying means 48 (SA7) selects, based on the set of physical information sent by one member through the member's terminal device 10n, the set of medical information pre-stored in the server 14 and related to the set of physical information, and sends the selected set of medical information to the member; and the information storing means 44 (SA6) stores the set of physical information of the member inputted through the member's terminal device 10n and the set of medical information supplied to the member. Thus, the server can easily store the respective sets of physical information, and the respective sets of medical information, of the members, on one hand, and, when each member requests a set of instructive information, the server can send, to the appropriate instructor, the set of medical information (including the set of physical information) stored by the information storing means 44, on the other hand. Thus, the instructor can make a more appropriate set of instructive information for the member and the server can supply the set of instructive information to the member.

In the illustrated embodiment, the diagnosing means 46 (or the diagnosing step SA5) automatically makes a diagnosis on a physical condition of the member, based on the set of physical information of the member inputted through the member's terminal device 10n; and the information sending means 62 or the information sending step SB3 sends the set of medical information including the diagnosis made by the diagnosing means 46. That is, when each member inputs his or her physical information to obtain his or her medical information from the server 14, the diagnosing means 46 automatically makes a diagnosis based on the physical information of the member, and this diagnosis is sent with the set of medical information to an appropriate instructor at the information sending step. Thus, the instructor can make a more appropriate set of instructive information for the member, and the server can supply the set of instructive information to the member.

In addition, in the illustrated embodiment, the instructor selecting means 60 or the instructor selecting step SB2 selects, from the pre-registered instructors, an appropriate instructor who corresponds to the sort of the instructive information requested by each member, i.e., is specialized in the sort. Thus, a more appropriate set of instructive information is supplied to the member.

While the present invention has been described in its embodiment by reference to the drawings, it is to be understood that the present invention can otherwise be embodied.

For example, in the illustrated embodiment, the server 14 is described such that the server 14 is provided by a single computer. However, the server may be provided by a plurality of computers, and those computers may be provided at respective positions remote from each other.

In the illustrated embodiment, each of the physical-information obtaining devices 18n connected to the member's terminal devices 10n has the functions of obtaining blood pressure BP, weight W, heart rate HR, electrocardiograph waveform ECG, temperature TB, autonomic-nerve activity, pulse-wave propagation velocity PWV, augmentation index AI, etc. However, each physical-information obtaining device 18n may be one that can obtain only a portion (one, two, ..., but not all) of the above-indicated sorts of physical information, or one that can obtain other sorts of physical information. The sorts of physical information that are not directly detected by each physical-information obtaining device 18n may be manually inputted through each member's terminal device 10n after having been measured using scales, a sphygmomanometer, a heart-rate meter, an electrocardiograph, etc. In this sense, each physical-information obtaining device 18n may be one that does not include any sensors.

In the flow charts shown in Figs. 4 and 5, the order of the steps may be changed, as needed. In addition, SA5 to SA11 of Fig. 4 may be omitted.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A method of supplying, from a server which is connected via a communication line to a plurality of member's terminal devices which are respectively operated by a plurality of members who are registered in advance in the server, a set of instructive information based on a set of medical information related to one of the members and stored in the server, to said one member, in response to an instructive-information request sent to the server from said one member, the method comprising:
an instructive-information-request identifying step of identifying said instructive-information request sent to the server from said one member,
an instructor selecting step of selecting, when said instructive-information request sent from said one member has been identified, one of a plurality of instructors who. are registered in advance in the server, and
an information sending step of sending, to the selected instructor, the set of medical information related to said one member and stored in the server, to request the instructor to make the set of instructive information for said one member.

2. A method according to claim 1, further comprising
a medical-information supplying step of selecting, from a plurality of sets of medical information respectively related to the plurality of members and stored in the server, the set of medical information related to a set of physical information of said one member inputted through a corresponding one of the member's terminal devices, and sending the selected set of medical information, to said one member, and
an information storing step of storing the set of physical information of said one member inputted through said one member's terminal device and adding the set of physical information to the selected set of medical information.

3. A method according to claim 1 or claim 2, further comprising a diagnosing step of making a diagnosis about a physical condition of said one member based on the set of physical information of said one member sent to the server from said one member's terminal device, wherein the information sending step comprises sending, to the selected instructor, the selected set of medical information including the made diagnosis.

4. A method according to any one of claims 1 to 3, wherein the instructor selecting step comprises selecting said one of the instructors registered in advance in the server, based on a sort of the instructive information requested by said one member.

5. A medical-information supplying apparatus including a server (14) which is connected via a communication line (16) to a plurality of member's terminal devices (10) which are respectively operated by a plurality of members who are registered in advance in the server, and supplying, from the server, a set of instructive information based on a set of medical information related to one of the members and stored in the server, to said one member, in response to an instructive-information request sent to the server from said one member, the apparatus comprising:
an instructive-information-request identifying means (58) for identifying said instructive-information request sent to the server (14) from said one member;
an instructor selecting means (60) for selecting, when said instructive-information-request identifying means has identified said instructive-information request sent from said one member, one of a plurality of instructors who are registered in advance in the server; and
an information sending means (62) for sending, to the instructor selected by the instructor selecting means, the set of medical information related to said one member and stored in the server, to request the instructor to make the set of instructive information for said one member.

6. An apparatus according to claim 5, further comprising:
a medical-information supplying means (48) for selecting, from a plurality of sets of medical information respectively related to the plurality of members and stored in the server, the set of medical information related to a set of physical information of said one member inputted through a corresponding one of the member's terminal devices (12n), and sending the selected set of medical information, to said one member; and
an information storing means (44) for storing the set of physical information of said one member inputted through said one member's terminal device (10) and adding the set of physical information to the selected set of medical information.

7. An apparatus according to claim 5 or claim 6, further comprising a diagnosing means (46) of making a diagnosis about a physical condition of said one member based on the set of physical information of said one member sent from said one member's terminal device (10), wherein the information sending means (62) sends, to the selected instructor, the selected set of medical information including the made diagnosis.

8. An apparatus according to any one of claims 5 to 7, wherein the instructor selecting means (60) selects said one of the. instructors registered in advance in the server, based on a sort of the instructive information requested by said one member.

9. An apparatus according to any one of claims 5 to 8, further comprising a plurality of physical-information obtaining devices (18) which are connected to the member's terminal devices (10), respectively, and which obtain respective sets of physical information from the members and supply the respective sets of physical information to the member's terminal devices.
